# EUROPEAN PATENT SPECIFICATION

(11) **EP 4 166 676 B1**
(45) Date of publication and mention of the grant of the patent: **30.07.2025**
(21) Application number: 21821235.5
(22) Date of filing: 11.06.2021
(51) Int. Cl.: C12Q 1/04, C12N 15/12, G01N 33/543, G01N 33/68

(54) **TECHNIQUE FOR CONTROLLING QUALITY OF HUMAN PLURIPOTENT STEM CELLS USING CULTURE BROTH**
VERFAHREN ZUR QUALITÄTSKONTROLLE VON MENSCHLICHEN PLURIPOTENTEN STAMMZELLEN MITHILFE VON KULTURBRÜHE
TECHNIQUE DE CONTRÔLE DE LA QUALITÉ DES CELLULES SOUCHES PLURIPOTENTES HUMAINES UTILISANT UN BOUILLON DE CULTURE

(30) Priority: 12.06.2020 JP 2020102063
(43) Date of publication of application: 19.04.2023
(73) Proprietor: National Institute of Advanced Industrial Science and Technology, Chiyoda-ku Tokyo 100-8921 (JP)
(72) Inventor: WATANABE, Tomoko, Tsukuba-shi, Ibaraki 305-8560 (JP); TATENO, Hiroaki, Tsukuba-shi, Ibaraki 305-8560 (JP); MAWARIBUCHI, Shuuji, Tsukuba-shi, Ibaraki 305-8560 (JP); HARAMOTO, Yoshikazu, Tsukuba-shi, Ibaraki 305-8560 (JP)
(74) Representative: Hoffmann Eitle
(86) International application number: PCT/JP2021/023128
(87) International publication number: WO 2021/251507

(56) References cited:
- JP-A- 2017 184 713
- US-A1- 2019 270 965
- MCILHINNEY R A J ET AL: "Effects of 12-O-tetradecanylphorbol 13-acetate (TPA) on a clonal human teratoma-derived embryonal carcinoma cell line", EXPERIMENTAL CELL RESEARCH, ELSEVIER, AMSTERDAM, NL, vol. 144, no. 2, 1 April 1983 (1983-04-01), pages 297 - 311, XP024792230, ISSN: 0014-4827, [retrieved on 19830401], DOI: 10.1016/0014-4827(83)90409-3
- TAYLOR-WEINER HERMES, SCHWARZBAUER JEAN E, ENGLER ADAM J: "Defined extracellular matrix components are necessary for definitive endoderm induction ", STEM CELLS, vol. 31, 1 October 2013 (2013-10-01), pages 2084 - 2094, XP055887429
- WATANABE TOMOKO; SAITO SAYOKO; HIEMORI KEIKO; KIYOI KAYO; MAWARIBUCHI SHUUJI; HARAMOTO YOSHIKAZU; TATENO HIROAKI: "SSEA-1-positive fibronectin is secreted by cells deviated from the undifferentiated state of human induced pluripotent stem cells", BIOCHEMICAL AND BIOPHYSICAL RESEARCH COMMUNICATIONS, ELSEVIER, AMSTERDAM NL, vol. 529, no. 3, 15 July 2020 (2020-07-15), Amsterdam NL , pages 575 - 581, XP086230522, ISSN: 0006-291X, DOI: 10.1016/j.bbrc.2020.06.074
- WATANABE TOMOKO; TATENO HIROAKI: "Elimination of cells deviated from human induced pluripotent stem cells with a photoactivatable IR700-labelled antibody", BIOCHEMICAL AND BIOPHYSICAL RESEARCH COMMUNICATIONS, ELSEVIER, AMSTERDAM NL, vol. 554, 25 March 2021 (2021-03-25), Amsterdam NL , pages 13 - 18, XP086540939, ISSN: 0006-291X, DOI: 10.1016/j.bbrc.2021.03.078

## Description

### Technical Field

The present invention relates to a method for determining or evaluating the undifferentiated state of human pluripotent stem cells using a culture supernatant, a method for controlling the quality of human pluripotent stem cells, and to the use of a kit in these methods.

### Background Art

Regenerative medicine involves transplanting pluripotent stem cells collected from oneself or another person or cells differentiated from these cells into tissues or organs with impaired functions to compensate for such tissues or organs, thereby reconstructing the tissues or regenerating the organs. In recent years, great expectations have been placed on regenerative medicine capable of performing fundamental repairment and/or regeneration upon treatment of diseases against which only symptomatic treatment such as drug treatment etc., is available.

Pluripotent stem cells such as embryonic stem cells (ES cells) and induced pluripotent stem cells (iPS cells) retain the undifferentiated state and can be induced to differentiate into various cells (e.g., hepatocytes, myocardial cells, etc.). Hence, pluripotent stem cells are expected to be a source of cells for transplantation in regenerative medicine. However, for actual use of pluripotent stem cells in medical care, it is essential to be able to reproducibly prepare cells for transplantation of the same quality from human pluripotent stem cells. For this purpose, it is important that the quality of human pluripotent stem cells being a cell source should be stable (in other words, the undifferentiated state should be maintained). However, maintaining the undifferentiated state is not easy since the properties of human pluripotent stem cells are easily changed by passage or manipulation. Therefore, it is extremely important to determine or evaluate the undifferentiated state of human pluripotent stem cells in controlling the quality.

Conventionally, the undifferentiated state of human pluripotent stem cells has been determined or evaluated by finding cells with changed morphology and in an advanced differentiation state from among cells observed under a microscope. However, in this case, it is not easy to find cells in an advanced differentiation state, and it is difficult to quantitatively evaluate how much such cells are mixed in.

In addition, markers indicating the undifferentiated state of human pluripotent stem cells (e.g., Oct3/4, rBC2-LCN, SSEA-4, Nanog, SOX2, c-Myc, Klf4, Lin28, TRA-1-60, TRA-1-81 etc.) and markers whose expression is observed in highly differentiated cells (e.g., SSEA-1 etc.) are known (Non Patent Literature 1 and 2). The presence or absence of the expression of these markers is confirmed by using a flow cytometer or immunostaining etc., and then the undifferentiated state of human pluripotent stem cells can be evaluated. However, this case is problematic not only in that a complicated step of collecting cells is indispensable, but also in that valuable human pluripotent stem cells that should originally be used for transplantation therapy are used for tests for detecting a marker(s) expressed in human pluripotent stem cells.

Therefore, in this field, the establishment of a new technique that enables simple, efficient, and non-invasive evaluation of the undifferentiated state of human pluripotent stem cells has been eagerly desired.

### Citation List

### Non Patent Literature

Non Patent Literature 1: Stem Cell Res. 2009 Jul;3(1): 3-11
Non Patent Literature 2: Stem Cells. 2004;22(5): 659-68

McIlhinney RA et al, Experimental cell research vol.144, no.2, 1 April 1983, studies the response of a human embryonic carcinoma cell line to TPA.

JP2017 184713 to Sumitomo Bakelite discloses a method and a kit for evaluating and determining the cell differentiation potential and the quality of somatic cells, based on the determination of alpha2-6 sialic acid in fibronectin in the culture supernatant of the stem cells.

US 2019/270965 teaches SSEA1 and SSEA4 as cell markers for differentiation of pluripotent stem cells.

### Summary of Invention

The invention is defined in the appended claims.

### Technical Problem

An object of the present invention is to provide a novel method that enables solving the above problems of the conventional methods for determining or evaluating the undifferentiated state of human pluripotent stem cells, and to simply, efficiently, and non-invasively determine or evaluate the undifferentiated state of human pluripotent stem cells.

### Solution to Problem

As a result of intensive studies to solve the above problems, the present inventors have found that: a culture supernatant of human pluripotent stem cells including cells having a low undifferentiated level contains fibronectin or SSEA-1-positive fibronectin; and that the proportion of such cells having a low undifferentiated level correlates with the amount of fibronectin or SSEA-1-positive fibronectin in the culture supernatant. The present inventors have further found that through detection or measurement of fibronectin or SSEA-1-positive fibronectin contained in a culture supernatant of human pluripotent stem cells, the undifferentiated state of human pluripotent stem cells can be determined or evaluated; and that based on the determination or evaluation results, the quality of human pluripotent stem cells can be controlled. The present inventors have further found that through the removal of fibronectin-positive cells as a target, cells having a low undifferentiated level can be selectively removed and thus the quality of human pluripotent stem cells can be controlled.

This description claims priority to Japanese Patent Application No. 2020-102063.

### Advantageous Effects of Invention

According to the present invention, it is possible to provide a novel method that enables simple, efficient, and non-invasive determination or evaluation the undifferentiated state of human pluripotent stem cells.

More specifically, the present invention is to detect or measure fibronectin or SSEA-1-positive fibronectin contained in a culture supernatant of human pluripotent stem cells as a test sample, so as to determine or evaluate the undifferentiated state of human pluripotent stem cells based on the detection or the measurement result of fibronectin or SSEA-1-positive fibronectin. According to the present invention, the undifferentiated state can be simply determined or evaluated without reducing valuable human pluripotent stem cells, and, cells having a low undifferentiated level can be selectively removed, so that the quality of human pluripotent stem cells can be controlled.

### Brief Description of Drawings

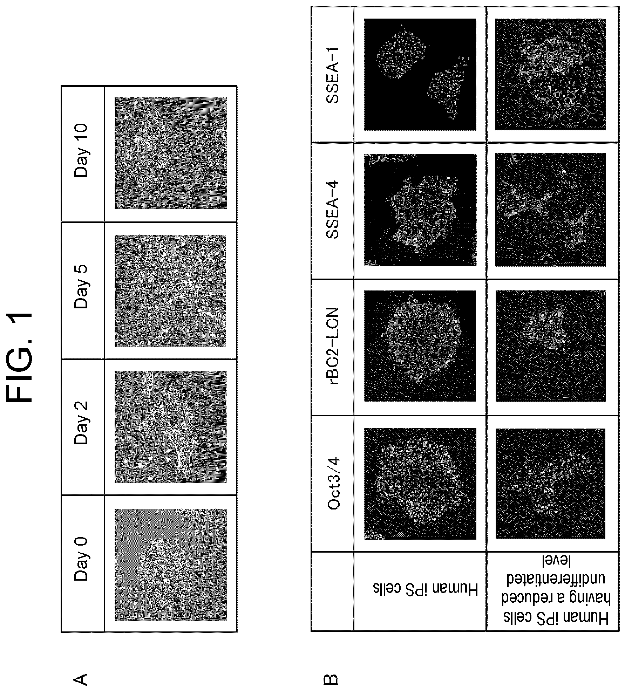

[FIG. 1] FIG. 1 depicts photographs, specifically, micrographs of human iPS cells having a reduced undifferentiated level in the process of preparing the cells (day 0 to day 10). Fig. 1A depicts phase-contrast micrographs of human iPS cells having a reduced undifferentiated level in the process of preparing the cells (day 0, day 2, day 5, and day 10). FIG. 1B depicts immunostaining images (fluorescence micrographs) of human iPS cells and human iPS cells having a reduced undifferentiated level immunostained with DAPI (blue) and Oct3/4, rBC2-LCN, SSEA-4, and SSEA-1 (green), respectively.

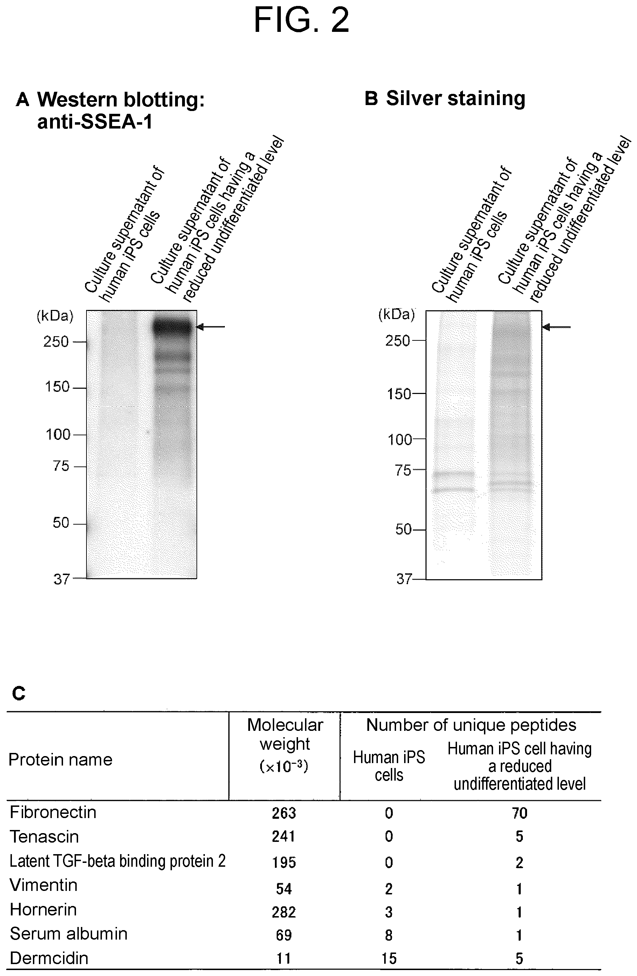

[FIG. 2] FIG. 2 depicts the results of analyzing SSEA-1-positive glycoprotein in a culture supernatant of human iPS cells having a reduced undifferentiated level. FIG. 2A is a photograph showing the results of immunoprecipitating each culture supernatant of human iPS cells and human iPS cells having a reduced undifferentiated level using rABA lectin and then subjecting the same to Western blotting using an anti-SSEA-1 monoclonal antibody. The region indicated by an arrow in a sample derived from the culture supernatant of human iPS cells having a reduced undifferentiated level indicates a > 250 kDa band. FIG. 2B is a photograph showing the results of subjecting each culture supernatant of human iPS cells and human iPS cells having a reduced undifferentiated level to immunoprecipitation using rABA lectin and then subjecting the same to silver staining. The region indicated by an arrow in a sample derived from the culture supernatant of human iPS cells having a reduced undifferentiated level indicates a > 250 kDa band. FIG. 2C depicts the results of analyzing proteins contained in the > 250 kDa band region excised from an SDS gel after silver staining depicted in FIG. 2B.

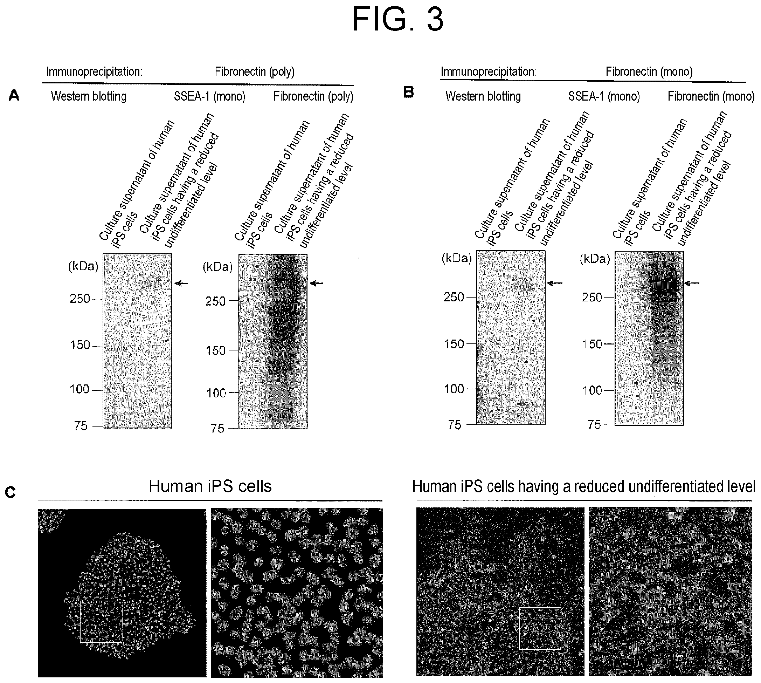

[FIG. 3] FIG. 3 depicts the results of analyzing SSEA-1-positive fibronectin in the culture supernatant of human iPS cells having a reduced undifferentiated level. FIG. 3A depicts photographs showing the results of subjecting each culture supernatant of human iPS cells and human iPS cells having a reduced undifferentiated level to immunoprecipitation using an anti-fibronectin polyclonal antibody, and then subjecting the same to Western blotting using the anti-SSEA-1 monoclonal antibody or the anti-fibronectin polyclonal antibody. FIG. 3B depicts photographs showing the results of subjecting each culture supernatant of human iPS cells and human iPS cells having a reduced undifferentiated level to immunoprecipitation using an anti-fibronectin monoclonal antibody, and then subjecting the same to Western blotting using the anti-SSEA-1 monoclonal antibody or the anti-fibronectin monoclonal antibody. FIG. 3C depicts photographs showing the immunostaining images (fluorescence micrographs) of human iPS cells (left) and human iPS cells having a reduced undifferentiated level (right) immunostained with DAPI (blue) and fibronectin (red). In each figure, an enlarged view of the area enclosed in a square is shown on the right.

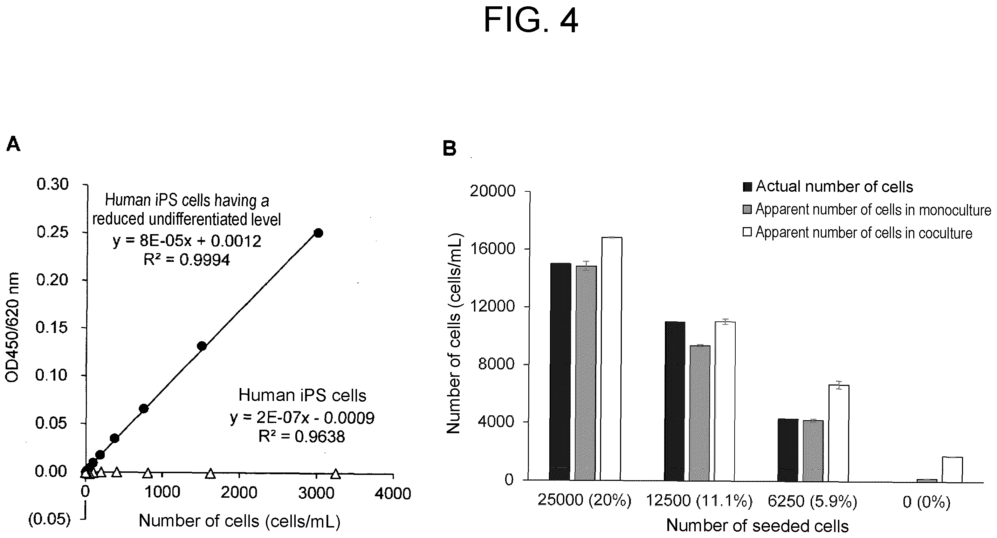

[FIG. 4] FIG. 4 depicts graphs showing the results of a sandwich ELISA assay using a fibronectin antibody and an SSEA-1 antibody. FIG. 4A depicts a calibration curve prepared by serially diluting each of culture supernatants obtained by culturing human iPS cells (white triangles) and human iPS cells having a reduced undifferentiated level (black circles) for 24 hours, respectively. FIG. 4B is a graph showing the results of sandwich ELISA using each of culture supernatants obtained by seeding human iPS cells having a reduced undifferentiated level at densities of 25,000 cells/mL, 12,500 cells/mL, 6,250 cells/mL, and 0 cells/mL, respectively. Black indicates the actual number of cells, gray indicates the apparent number of cells when human iPS cells having a reduced undifferentiated level were cultured alone, and white indicates the apparent number of cells when human iPS cells having a reduced undifferentiated level were co-cultured with human iPS cells.

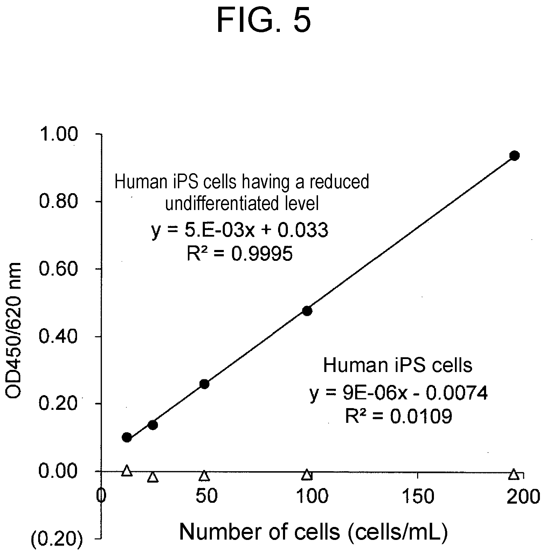

[FIG. 5] FIG. 5 is a graph showing the results of a sandwich ELISA assay using a fibronectin monoclonal antibody and a fibronectin polyclonal antibody. FIG. 5 depicts a calibration curve prepared by serially diluting each of the culture supernatants obtained by culturing human iPS cells (white triangles) and human iPS cells having a reduced undifferentiated level (black circles) for 24 hours.

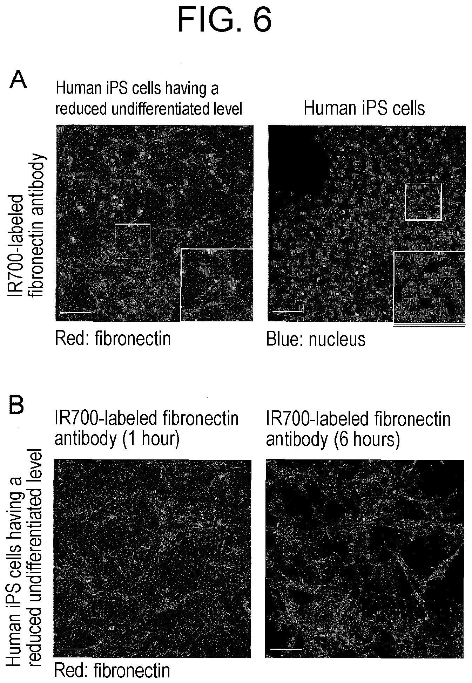

[FIG. 6] FIG. 6 depicts photographs showing the immunostaining images (fluorescence micrographs) obtained by immunostaining human iPS cells and human iPS cells having a reduced undifferentiated level with an IR700-labeled fibronectin antibody. FIG. 6A depicts immunostaining images of human iPS cells having a reduced undifferentiated level (left) and human iPS cells (control: right) immunostained with the IR700-labeled fibronectin antibody (red) and Hoechst 33342 (blue). An enlarged view of the area enclosed in a square is shown in a lower right frame. FIG. 6B depicts immunostaining images obtained by treating human iPS cells having a reduced undifferentiated level with the IR700-labeled fibronectin antibody (red) for 1 hour (left) and 6 hours (right). In each figure, the scale bar indicates 100 µm.

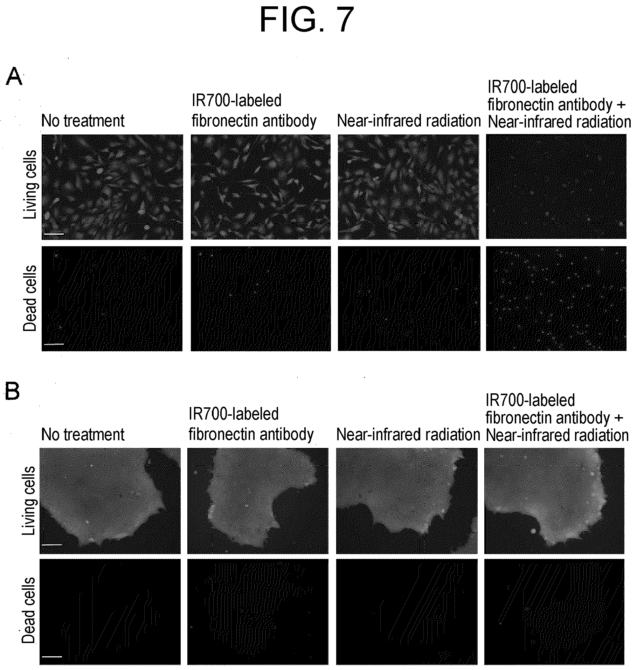

[FIG. 7] FIG. 7 depicts photographs showing the results of analyzing the cell viability of each cell type (fluorescence micrographs) after treatment of human iPS cells and human iPS cells having a reduced undifferentiated level with the IR700-labeled fibronectin antibody, followed by near-infrared (NIR) radiation. FIG. 7A depicts the results of subjecting human iPS cells having a reduced undifferentiated level to no treatment, treatment with the IR700-labeled fibronectin antibody, NIR radiation, and treatment with the IR700-labeled fibronectin antibody followed by NIR radiation, respectively, and then analyzing the cell viability by living cell staining (calcein-acetoxymethyl (AM): upper (green)) and dead cell staining (EthD-1: lower (red)). FIG. 7B depicts the results of analyzing the cell viability of human iPS cells in the same manner. In each figure, the scale bar indicates 100 µm.

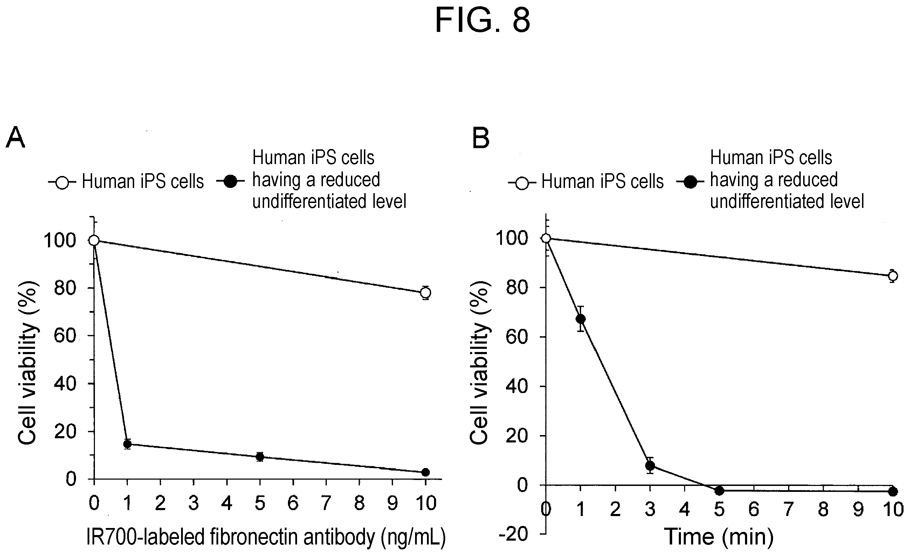

[FIG. 8] FIG. 8 depicts graphs showing the results of analyzing the quantitative effects of treatment with the IR700-labeled fibronectin antibody followed by NIR radiation on the cell viability of human iPS cells and human iPS cells having a reduced undifferentiated level. FIG.8A is a graph showing the cell viability (%) of human iPS cells (white circles) and human iPS cells having a reduced undifferentiated level (black circles) treated with an IR700-labeled fibronectin antibody at predetermined concentrations (0 to 10 ng/mL) and then subjected to NIR radiation. The cell viability (%) is a value relative to the absorbance at 450 nm of untreated cells designated as 100%, after NIR radiation, addition of a CCK-8 solution to the medium and then measurement of the absorbance at 450 nm, and is the average value in triplicate measurements ± standard deviation. FIG. 8B is a graph showing the cell viability (%) of human iPS cells (white circles) and human iPS cells having a reduced undifferentiated level (black circles) subjected to treatment with the IR700-labeled fibronectin antibody followed by NIR radiation for a predetermined time (0 to 10 minutes). The cell viability (%) is a value relative to the absorbance at 450 nm of cells treated with IR700-labeled fibronectin antibody alone designated as 100% after NIR radiation, addition of a CCK-8 solution to the medium, and is the average value in triplicate measurements ± standard deviation.

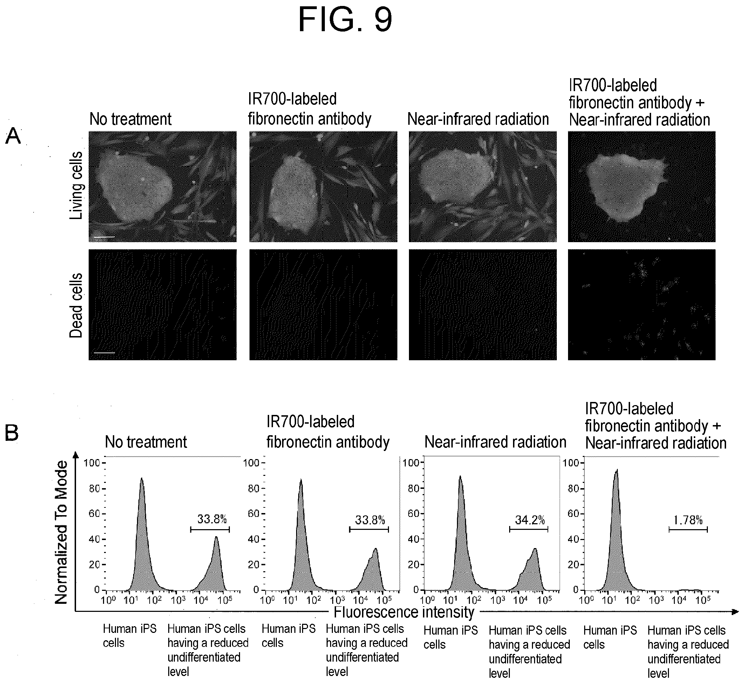

[FIG. 9] FIG. 9 depicts the experimental results of selective removal of human iPS cells having a reduced undifferentiated level using treatment with an IR700-labeled fibronectin antibody followed by NIR radiation in the coexistence of human iPS cells. FIG. 9A depicts photographs showing the results of subjecting the co-culture of human iPS cells and human iPS cells having a reduced undifferentiated level to no treatment, treatment with the IR700-labeled fibronectin antibody, NIR radiation, and treatment with the IR700-labeled fibronectin antibody followed by NIR radiation, respectively, and then analyzing the cell viability of each cell type (fluorescence micrographs) by living cell staining (calcein-acetoxymethyl (AM): upper (green)) and dead cell staining (EthD-1: lower (red)). The scale bar indicates 100 µm. FIG. 9B depicts the results of subjecting the co-culture of human iPS cells and human iPS cells having a reduced undifferentiated level stained with CellTrackerGreen-5-chloromethylfluorescein diacetate to no treatment, treatment with the IR700-labeled fibronectin antibody, NIR radiation, and treatment with the IR700-labeled fibronectin antibody followed by NIR radiation, respectively, collecting, and then measuring the proportion of each cell type by a flow cytometer.

### Description of Embodiments

### 1. Undifferentiated state of human pluripotent stem cells

In the present invention, the term "pluripotent" refers to the capability of differentiating into cells of any lineage of three germ layers (endoderm, mesoderm, ectoderm) (but unable to differentiate into blastocyst). Namely, it means "pluripotency."

The term "human pluripotent stem cells" refers to human cells being pluripotent and potentially capable of differentiating into various tissues of a living body including three germ layers (endoderm, mesoderm, ectoderm). Examples of such cells include human embryonic stem cells (ES cells), human induced pluripotent stem cells (iPS cells), and cells having pluripotency equivalent thereto. Preferably, in the present invention, the "human pluripotent stem cells" are human ES cells or human iPS cells, and more preferably human iPS cells.

The term "human iPS cells" refers to induced pluripotent stem cells that can be produced by introducing specific factors known as nuclear reprogramming factors (e.g., Oct3/4, Sox2, Klf4, c-Myc, Nanog, Lin28, L-Myc, hTERT, SV40 large T, etc.) into human somatic cells or undifferentiated stem cells for reprogramming (initialization) these cells (Takahashi K. et al., Cell (2007) 131, 861-872; Okita, K. et al., Nature (2007) 448, 313-317;Nakagawa M. et al., Nature Biotechnology, (2008)26, 101-106;Yu J. et al., Science (2007)318, 1917-1920;Park IH. et al., Nature (2007) 451, 141-146 etc.).

In the present invention, "human iPS cells" may be those produced from human somatic cells or undifferentiated stem cells according to conventionally known means, or established human iPS cell lines (e.g., 201B7 cell line, 253G1 cell line, 409B2 cell line, 454E2 cell line, 606A1 cell line, 610B1 cell line, 648A1 cell line, Ff-WJ-18 cell line, Ff-I01s01 cell line, Ff-I01s02 cell line, Ff-I01s04 cell line, Ff-I01s06 cell line, Ff-I14s03 cell line, Ff-I14s04 cell line, QHJI01s01 cell line, QHJI01s04 cell line, QHJI14s03 cell line, QHJI14s04 cell line, 253G1 cell line, HiPS-RIKEN-1A cell line, HiPS-RIKEN-2A cell line, HiPS-RIKEN-12A cell line, Nips-B2 cell line, etc., (examples thereof are not limited thereto)).

As used herein, the term "undifferentiated state" of human pluripotent stem cells refers to a state in which human pluripotent stem cells retain pluripotency and have not differentiated (also referred to as "deviated"). On the other hand, when differentiation (deviation) occurs in human pluripotent stem cells, pluripotency decreases or disappears, and accordingly, the level of "undifferentiated state" (or the undifferentiated level) is reduced or disappears. In human pluripotent stem cells retaining the undifferentiated state, the expression of one or more undifferentiation marker genes and/or proteins selected from the group consisting of Oct3/4, rBC2-LCN, SSEA-4, Nanog, SOX2, c-Myc, Klf4, Lin28, TRA-1-60, TRA-1-81 etc., is generally observed. On the other hand, in cells whose undifferentiated level has been reduced or disappeared, the expression of the above undifferentiation marker gene(s) and/or protein(s) is decreased or disappears as compared with human pluripotent stem cells retaining the undifferentiated state, and instead, the expression of differentiation markers, fibronectin gene and/or protein such as SSEA-1, vimentin, N-cadherin, Twist, Zeb1/2, Snail, Slug, etc., is observed.

As used herein, the term "human pluripotent stem cells (or human iPS cells) having a low undifferentiated level", "human pluripotent stem cells (or human iPS cells) having a reduced undifferentiated level" or "the undifferentiated level of human pluripotent stem cells (or human iPS cells) has been reduced" refers to a population of human pluripotent stem cells (or human iPS cells) including the cells whose undifferentiated level has been reduced or disappeared. That is, "human pluripotent stem cells (or human iPS cells) having a low undifferentiated level", "human pluripotent stem cells (or human iPS cells) having a reduced undifferentiated level", or "the undifferentiated level of human pluripotent stem cells (or human iPS cells) has been reduced" can include a form in which human pluripotent stem cells (or human iPS cells) retaining the undifferentiated state and cells whose undifferentiated level has been reduced or disappeared coexist.

### 2. Determination or evaluation of undifferentiated state of human pluripotent stem cells

### (2-1) Fibronectin

"Fibronectin" is a large secretory glycoprotein known as a cell adhesion molecule, which is known to be synthesized in various cells and secreted extracellularly (Gene ID: 2335 in the NCBI database). Fibronectin is known to have many functions that support the normal vital functions of spinal functions, such as adhesion to extracellular matrix, formation and retention of connective tissue, wound healing, and formation and maintenance of tissue and organ morphologies and compartments during embryogenesis. Fibronectin (monomer) is a soluble glycoprotein having a molecular weight of 210 to 250 kDa (2,146 to 2,325 amino acid residues), and is known to have seven N-type sugar chains and one O-type sugar chain. In the present invention, examples of the "fibronectin" may include the following "SSEA-1 positive fibronectin".

### (2-2) SSEA-1-positive fibronectin

In the present invention, the term "SSEA-1 positive fibronectin" refers to a glycoprotein having secretory fibronectin, to which SSEA-1 is bound, as a core protein.

"SSEA-1 (Stage-specific Embryonic Antigen-1)" is a sugar chain marker having Lewis X, that is, Galβ1-4 (Fucα1-3) GlcNAc as a sugar chain antigen (epitope). It has been reported that SSEA-1 is not expressed in human pluripotent stem cells retaining the undifferentiated state, and its expression on the cell membrane increases with differentiation (Thomson JA. et al., Science. 1998 Nov 6; 282 (5391): 1145-7.). SSEA-1 is considered to be bound to the non-reducing end of one or more sugar chains of seven N-type sugar chains and one O-type sugar chain of fibronectin.

### (2-3) Culture supernatant

In the present invention, fibronectin or SSEA-1 positive fibronectin is detected or measured using a culture supernatant of an *in vitro* culture of human pluripotent stem cells as a test sample. By using a culture supernatant, the undifferentiated state can be determined or evaluated non-invasively without using valuable human pluripotent stem cells that should originally be used for transplantation therapy. In a culture step, a culture supernatant is generally replaced with a fresh culture supernatant at regular intervals. Therefore, fibronectin or SSEA-1 positive fibronectin is detected or measured after a detectable amount of fibronectin or SSEA-1 positive fibronectin is secreted into the culture supernatant after replacement. The time required for a detectable amount of fibronectin or SSEA-1-positive fibronectin to be secreted in a culture supernatant after replacement of the culture supernatant may vary depending on the type and amount of cells and the culture conditions. Therefore, the time required for collecting a culture supernatant to be used for detecting or measuring fibronectin or SSEA-1 positive fibronectin after replacement of the culture supernatant can be appropriately set depending on the cell type and culture conditions, but ranges from, for example, about 18 to 72 hours. Since medium exchange is usually performed about every 1 to 3 days, it is preferable to use a culture supernatant to be discarded at the time of medium exchange. A culture supernatant collected using a conventionally known solid-liquid separation method (e.g., centrifugation, filtration, etc.) is preferably used as a culture supernatant.

### (2-4) Probe

### (2-4-1) Probe that recognizes fibronectin

In the present invention, examples of a probe that can be used as a "probe that recognizes fibronectin" include proteins, polypeptides, and compounds capable of recognizing and binding to fibronectin. Preferably an anti-fibronectin antibody or a fragment thereof that recognizes fibronectin as an antigen (epitope) can be used. The anti-fibronectin antibody can be prepared by a conventional method, and can be obtained, for example, by immunizing an animal with fibronectin or a fragment thereof as it is or with the same bound to a carrier protein such as albumin or KLH. Alternatively, the anti-fibronectin antibody may be any antibody capable of recognizing and binding to, and preferably specifically binding to fibronectin as an epitope. Examples of the anti-fibronectin antibody that can be used herein without particular limitation include a polyclonal antibody, a monoclonal antibody, a chimeric antibody, a humanized antibody, and a fully human antibody. Further, in the present invention, a fragment of the anti-fibronectin antibody can also be used as long as it is capable of recognizing and binding to, and preferably specifically binding to fibronectin as an epitope. Examples of such antibody fragments include Fab, Fab', F (ab')₂, Fv, scFv, dsFv, diabodies, sc (Fv)₂, etc. Polymers of these fragments (e.g., dimers, trimmers, tetramers, polymers etc.) can also be used in the present invention. Known or commercially available anti-fibronectin antibodies can also be used in the present invention.

One type of such probe that recognizes fibronectin may be used alone, or two or more, a plurality of types of probes may be used in combination.

### (2-4-2) Probe that recognizes SSEA-1 as epitope

SSEA-1 positive fibronectin in a culture supernatant can be detected or measured using (a) a probe that recognizes SSEA-1 as an epitope and (b) a probe that recognizes the fibronectin described above. The use of these two types of probes enables detection of a target protein with high specificity and high sensitivity, and thus is preferable.

In the present invention, examples of (a) "a probe that recognizes SSEA-1 as an epitope" that can be used herein include an SSEA-1 binding lectin that recognizes and binds to the sugar chain structure of SSEA-1 (e.g., Lotus agglutinin (Lotus Tetragolonobus lectin, LTL), DC-SIGN, lectin derived from *Pseudomonas aeruginosa (Pseudomonas aeruginosa* lectin, PA-IIL), etc. (but examples thereof are not limited thereto)), peptides, aptamers, and an anti-SSEA-1 antibody that recognizes SSEA-1 as a sugar chain antigen (epitope) or a fragment thereof. Preferably, in the present invention, the "probe that recognizes SSEA-1 as an epitope" is an anti-SSEA-1 antibody or a fragment thereof. The anti-SSEA-1 antibody can be prepared by a conventional method, for example, by immunizing an animal with SSEA-1 as it is or SSEA-1 bound to a carrier protein such as albumin or keyhole limpet hemocyanin (KLH). Alternatively, a known or commercially available anti-SSEA-1 antibody can also be used in the present invention. The anti-SSEA-1 antibody may be any antibody capable of recognizing and binding to, and preferably specifically binding to SSEA-1 as an epitope. Examples of the anti-SSEA-1 antibody that can be used without particular limitation include a polyclonal antibody, a monoclonal antibody, a chimeric antibody, a humanized antibody, a fully human antibody etc. Further, in the present invention, a fragment of the anti-SSEA-1 antibody can also be used as long as it is capable of recognizing and binding to, and preferably specifically binding to SSEA-1 as an epitope. As such an antibody fragment, those defined above can be used.

One type of such probe that recognizes SSEA-1 as an epitope may be used alone, or two or more, a plurality of types of such probes may be used in combination.

### (2-5) Detection or measurement of fibronectin or SSEA-1 positive fibronectin

Fibronectin or SSEA-1 positive fibronectin in a culture supernatant can be detected or measured by a technique that is generally employed for the detection or quantification of a protein in combination with the use of each of the above probes. For example, the detection or measurement can be performed using one or more of such techniques such as enzyme-linked immunosorbent assay (ELISA) method, a sandwich assay method, a competitive method, a Western blotting method, a chromatography method, immunoprecipitation etc. Preferably, fibronectin in a culture supernatant is detected or measured by a sandwich assay method using two or more types of probes that recognizes fibronectin, and more preferably, SSEA-1 positive fibronectin in a culture supernatant is detected or measured by the sandwich assay method using probes (a) and (b) above.

In the sandwich assay method, one of the probes to be used (hereinafter referred to as "first probe") is immobilized on a substrate. Examples of a substrate on which the first probe is immobilized include plates (e.g., microwell plate, etc.), microarray substrates (e.g., slide glass for microarray, etc.), tubes, beads (e.g., plastic beads, magnetic beads, etc.), and carriers for chromatography (e.g., Sepharose (Trade Mark), etc.), membranes (e.g., nitrocellulose membrane, PVDF membrane, etc.), and gels (e.g., polyacrylamide gel, etc.). Among them, plates, beads and membranes are preferably used, and plates are most preferably used because of their ease of handling.

The first probe can be immobilized on a substrate by immobilization using a biotin-avidin bond. Specifically, the first probe to be immobilized is biotinylated in advance and then the biotinylated probe can be prepared in a form immobilized on a streptavidin-coated substrate (well). In this case, the detection sensitivity is improved and the background can be significantly reduced.

Other immobilization methods include a physical adsorption method and a chemical bonding method. When the physical adsorption method is used, examples thereof include methods of activating the surface by γ-ray treatment, electron beam treatment, UV treatment, plasma treatment, corona treatment, etc. When the chemical bonding method is used, a substituted or unsubstituted phenol active ester group that is a p-nitrophenyl group or the like; and an N-hydroxyimide active ester group that is an N-hydroxysuccinimide group, an N-hydroxyphthalimide group, an N-hydroxy-5-norbornen-2,3-dicarboxyimide or the like, a carboxyl group, a carbonyl group, an aldehyde group, an epoxy group, an amino group, a thiol group, a maleimide group or the like, are preferably present on the surface of the substrate.

A culture supernatant, which is a test sample, is diluted or not diluted with a buffer solution, and then added to a well in which the first probe has been immobilized, for interaction. Non-specifically bound contaminants are washed off with a buffer solution.

Next, a buffer solution containing a probe different from the first probe (hereinafter referred to as "second probe"), which has been indirectly or directly labeled, is added for reaction. Examples of a label for the second probe include, but are not limited to, labeling substances such as fluorescent substances (e.g., FITC, rhodamine, Cy3, Cy5, etc.), radioactive substances (e.g., 13C, 3H, etc.), and enzymes (e.g., alkaline phosphatase, peroxidase (horseradish peroxidase, etc.), glucose oxidase, β-galactosidase, etc.). Alternatively, the second probe may be labeled with biotin and (strepto)avidin may be labeled with the above-mentioned labeling substance to utilize the binding between biotin and (strepto)avidin.

Further, as used herein, cases of "indirectly or directly labeled probe different from the first probe" or "labeled second probe" include a case in which the probe itself is not directly labeled, but the probe is indirectly labeled with a labeled second antibody that binds to the probe for detection.

When an enzyme is used as a labeling substance, detection is performed using an appropriate substrate according to the enzyme to be used. For example, when peroxidase is used as the enzyme, a substrate to be used herein is o-phenylenediamine (OPD), tetramethylbenzidine (TMB), 2,2'-azinobis [3-ethylbenzothiazolin-6-sulfonic acid] (ABTS), 3,3'-diaminobenzidine (DAB), Amplex (registered trademark) Red, 3-(p-hydroxyphenyl)propion (HPPA), luminol, or the like. When alkaline phosphatase is used, p-nitrophenyl phosphate (PNPP), 4-methylumbelliferyl phosphate, 5-bromo-4-chloro-3-indolyl phosphate (BCIP)/nitroblue tetrazolium chloride (NBT), AttoPhos (registered trademark), 4-methylumbelliferyl phosphate (4-MUP), ECF, DDAO phosphate, dioxetane, CDP-Star (trademark), AMPPD (registered trademark), CSPD (registered trademark) etc., are used. As an enzyme reaction stop solution and a substrate lysate, conventionally known ones can be appropriately selected and used according to the selected enzyme.

In the sandwich assay method, fibronectin or SSEA-1-positive fibronectin in a culture supernatant binds to the first probe immobilized on the substrate and also binds to the labeled second probe to form a complex. Subsequently, a signal generated from the labeled second probe in this complex is detected or measured, so that fibronectin or SSEA-1-positive fibronectin in the sample can be detected or measured. The signal can be detected or measured using an appropriate detection or measurement device depending on the labeling substance used.

Examples of a method for detecting or measuring the signal, which can be used herein, include ELISA, immunochromatography, radioimmunoassay (RIA), fluorescence immunoassay (FIA method), chemiluminescent immunoassay, and an evanescent wave analysis method. These methods are known to those skilled in the art, and any method may be selected. In addition, these methods may be performed according to ordinary procedures, and the actual setting of reaction conditions etc., are within the technical scope normally available to those skilled in the art. In the present invention, signal detection or measurement is preferably carried out by the sandwich ELISA assay method that involves detecting or measuring a signal generated from a second probe labeled with an enzyme such as horseradish peroxidase.

For the quantitative measurement of fibronectin or SSEA-1-positive fibronectin in a culture supernatant, for example, the above sandwich assay method is performed using recombinant fibronectin expressed by human cells, or, fibronectin or SSEA-1-positive fibronectin purified from a culture supernatant of cells expressing the fibronectin or SSEA-1-positive fibronectin, as a standard substance, and then the amount of the labeled signal corresponding to the amount of the standard substance can be detected and blotted to prepare a calibration curve. Therefore, the amount of the labeled signal can be converted into the amount of the fibronectin or SSEA-1 positive fibronectin, and then the fibronectin or SSEA-1 positive fibronectin in the culture supernatant can be quantitatively measured. Furthermore, with the use of a culture supernatant of human pluripotent stem cells having a low undifferentiated level, a culture supernatant of recombinant cells into which a gene encoding fibronectin has been introduced, a culture supernatant of recombinant cells into which a gene encoding Fut9 for Lewis X synthesis and a gene encoding fibronectin have been introduced, a culture supernatant of fibronectin-expressing cells, or a culture supernatant of SSEA-1 positive fibronectin-expressing cells, or fibronectin or SSEA-1-positive fibronectin purified from such a culture supernatant, as a standard substance, the amount of the labeled signal corresponding to a predetermined number of cells can be detected and blotted by the above sandwich assay method to prepare a calibration curve. Therefore, the amount of the labeled signal can be converted into the number of cells expressing fibronectin or SSEA-1 positive fibronectin, and the cells expressing the fibronectin or SSEA-1 positive fibronectin during culture can be quantitatively measured.

### (2-6) Determination or evaluation of undifferentiated state

In the present invention, the undifferentiated state of human pluripotent stem cells can be determined or evaluated based on the above results of detecting or measuring fibronectin or SSEA-1 positive fibronectin in a culture supernatant of human pluripotent stem cells. The detection or measurement results of fibronectin or SSEA-1 positive fibronectin in a culture supernatant of human pluripotent stem cells correlates with the undifferentiated state of human pluripotent stem cells. When fibronectin or SSEA-1 positive fibronectin is detected in a culture supernatant, it can be determined and evaluated that the undifferentiated level of the pluripotent stem cells has been reduced, that is, cells whose undifferentiated level has been reduced or disappeared are included. In addition, it can be determined and evaluated that, the larger the amount of fibronectin or SSEA-1-positive fibronectin in a culture supernatant, the more reduced undifferentiated level of human pluripotent stem cells, that is, the more number of cells whose undifferentiated level has been reduced or disappeared are included.

### 3. Control of the quality of human pluripotent stem cells

In the present invention, the expression "controlling the quality of human pluripotent stem cells" means controlling the undifferentiated state of human pluripotent stem cells, which can be performed according to the determination or evaluation result of the undifferentiated state of human pluripotent stem cells. For example, when fibronectin or SSEA-1-positive fibronectin is detected in a culture supernatant, or a predetermined amount of fibronectin or SSEA-1-positive fibronectin is detected in a culture supernatant by the above method for determining or evaluating the undifferentiated state of human pluripotent stem cells, the human pluripotent stem cells in the culture are determined to be human pluripotent stem cells having a low undifferentiated level. Accordingly, the culture can be discarded, the human pluripotent stem cells can be collected from the culture, or cells having a low undifferentiated level can be removed from the culture, for example. Specifically, human pluripotent stem cells having a low undifferentiated level (or human pluripotent stem cells in an undifferentiated state) can be screened based on the determination or evaluation result of the undifferentiated state of human pluripotent stem cells. This enables the supply of human pluripotent stem cells having stable quality. For example, a criterion can be set for determining that "human pluripotent stem cells (or human iPS cells) having a low undifferentiated level", "human pluripotent stem cells (or human iPS cells) having a reduced undifferentiated level", or "the undifferentiated level of human pluripotent stem cells (or human iPS cells) has been reduced" means that the proportion of cells whose undifferentiated level has been reduced or disappeared accounts for 10% or more of the total number of cells. Based on this criterion, when the proportion of cells whose undifferentiated level has been reduced or disappeared accounts for less than 10% of the total number of cells, these cells can be supplied as human pluripotent stem cells having stable quality. However, the criterion can be set arbitrarily and is not limited to the above value.

Cells having a low undifferentiated level can be removed from the culture by isolating, from the culture, or killing cells expressing fibronectin (referred to as "fibronectin-positive cells") or cells expressing SSEA-1-positive fibronectin (referred to as "SSEA-1-positive fibronectin-positive cells"). In the present invention, the "fibronectin-positive cells" may include "SSEA-1-positive fibronectin-positive cells".

Fibronectin-positive cells or SSEA-1-positive fibronectin-positive cells can be isolated by a commonly employed technique for cell isolation or purification, for example, by one or more of chromatography, magnetic separation, centrifugation, flow cytometer, cell sorter (fluorescence activated cell sorter (FACS), etc.), etc. combined with the use of the above probes.

Fibronectin-positive cells or SSEA-1-positive fibronectin-positive cells can be killed using any means capable of selectively, preferably specifically, killing these cells. For example, a technique, so-called photoimmunotherapy, neutron capture therapy etc., that combines the use of the above probe(s) labeled with a predetermined photosensitizer with exposure to near infrared rays (wavelength 700-2,500 nm), ultraviolet rays (wavelength 10-400 nm), or thermal neutron rays can be used. Examples of a photosensitizer include a light-absorbing phthalocyanine dye IRDye700Dx (IR700), boron 10 (¹⁰B), cadmium selenide (CdSe), etc., that is activated by near infrared radiation. In one embodiment of the present invention, the probe labeled with IR700 is bound to target fibronectin-positive cells or SSEA-1-positive fibronectin-positive cells, and then exposed to near infrared rays; the probe labeled with ¹⁰B is bound to target fibronectin-positive cells or SSEA-1-positive fibronectin-positive cells, and then exposed to thermal neutron rays; or the probe labeled with CdSe is bound to target fibronectin-positive cells or SSEA-1-positive fibronectin-positive cells, and then exposed to ultraviolet rays, so that the fibronectin-positive cells or the SSEA-1-positive fibronectin-positive cells, to which the probe has been bound, can be selectively and preferably specifically killed. Alternatively, a drug delivery system using the above probe, to which an inhibitor of a predetermined factor of cells or a drug promoting apoptosis (e.g., an anticancer drug, a molecular target drug, etc.) has been bound, can be used. The probe, to which a predetermined inhibitor or a drug has been bound, is bound to target fibronectin-positive cells or SSEA-1-positive fibronectin-positive cells, to deliver the inhibitor or drug to the cells and to cause the same to act on the cells, so that the cells can be killed selectively, and preferably killed specifically.

### 4. Kit

In the present invention, the kit is used in the above method for determining or evaluating the undifferentiated state of human pluripotent stem cells, or in the above method for controlling the quality of human pluripotent stem cells. The kit comprises one or more probes that recognize fibronectin, which are used to detect or measure fibronectin in a culture supernatant.

Here, when "fibronectin" is SSEA-1 positive fibronectin, the above probes include (a) a probe that recognizes SSEA-1 as an epitope and (b) a probe that recognizes fibronectin.

All of the probes included in the kit described herein are as defined above.

In one embodiment, the above probes included in the kit described herein include the first probe immobilized on a substrate and the labeled second probe. For example, the kit comprises an anti-fibronectin monoclonal antibody or a fragment thereof immobilized on a substrate as the first probe, and a labeled anti-fibronectin polyclonal antibody or a fragment thereof as the second probe. Alternatively, the kit comprises an anti-fibronectin polyclonal antibody or a fragment thereof immobilized on a substrate, and a labeled anti-fibronectin monoclonal antibody or a fragment thereof. Further, for example, the kit comprises (a) a probe that recognizes SSEA-1 as an epitope, which is immobilized on a substrate, as the first probe, and (b) a labeled probe that recognizes fibronectin, as the second probe, or, comprises (a) a labeled probe that recognizes SSEA-1 as an epitope and (b) a probe that recognizes fibronectin, which is immobilized on a substrate.

Furthermore, the kit described herein can include recombinant fibronectin that can be used as a standard substance and is expressed in human cells, human pluripotent stem cells having a low undifferentiated level, and recombinant cells into which a gene encoding fibronectin has been introduced, recombinant cells into which a gene encoding Fut9 for Lewis X synthesis and a gene encoding fibronectin have been introduced, a culture supernatant of fibronectin-expressing cells, a culture supernatant of SSEA-1-positive fibronectin-expressing cells, fibronectin or SSEA-1 positive fibronectin purified from the culture supernatant, etc., which can be used to prepare a calibration curve for quantitative measurement of fibronectin or SSEA-1 positive fibronectin and cells expressing the same.

Further, when the kit described herein is a kit for use in a method for controlling the quality of human pluripotent stem cells, such a kit can further include a means for removing cells having a low undifferentiated level from the culture. Examples of such a means for removing cells having a low undifferentiated level include, but are not limited to, a chromatographic column filled with one or more probes that recognize fibronectin, which are bound to an arbitrary carrier, beads for magnetic separation, wherein the probe is bound to magnetic beads, and the probes labeled with photosensitizers, such as IR700, ¹⁰B, and CdSe, etc. When the "fibronectin" is an SSEA-1-positive fibronectin, the above probes include (a) a probe that recognizes SSEA-1 as an epitope and (b) a probe that recognizes fibronectin.

Further, the kit described herein may include containers for reagents, a package, an instruction manual, etc., as well as a measuring device, and a light source for irradiation with near-infrared rays, ultraviolet rays, or thermal neutron rays, etc.

Hereinafter, the present invention will be described in more detail with reference to Examples.

### Examples

### Example 1: Preparation of human iPS cells having a reduced undifferentiated level

Human iPS cells, 201B7 (HPS0063), were obtained from RIKEN BioResource Research Center. Human iPS cells were cultured on Matrigel (Catalog No. 354230; BD Biosciences) using mTeSR1 medium (Catalog No. ST-05850; Stem Cell Technologies) under feeder-free conditions. Medium exchange was performed daily.

Human iPS cells having a reduced undifferentiated level were prepared by the following method. Human iPS cells were subcultured at a low density (1:15 to 1:20). A supplement was incubated at 56°C for 30 minutes to inactivate it, and then added to an mTeSR1 medium. The cells were cultured at 37°C and 5% CO₂. Medium exchange was performed every 3 to 4 days, and the cells were cultured for about 2 weeks. When the cell density reached 80% on day 8 of culture, rBC2LCN-PE38 (Tateno et al., Stem Cell Reports 2015) was added at a concentration of 100 ng/mL. Note that in Examples, cells were always cultured in an environment of 37°C and 5% CO₂.

FIG. 1A depicts phase-contrast micrographs in the process of preparing human iPS cells having a reduced undifferentiated level. It was observed that the morphology of the cell colonies gradually changed during the culture process and the cell-cell adhesion was loosened. Specifically, on day 2 of culture, thorn-shaped portions appeared on the borders of the colonies, on day 5 of culture, the cell-cell adhesion was loosened to form gaps, and on day 10 of culture, large and flat cell populations were observed. From this result, it was determined that the human iPS cells subjected to the treatment with rBC2LCN-PE38 were cells having a low undifferentiated level.

Human iPS cells (control) and human iPS cells having a reduced undifferentiated level were each fixed with 4% paraformaldehyde (Catalog No. 163-20145; Wako) for 15 minutes at room temperature and rinsed with PBS. Transcription factor staining was performed by immersing cell samples in PBS containing 0.4% Triton X-100 (Catalog No. T8787; Sigma) for 10 minutes. The cells were pre-hybridized in PBS containing a blocking reagent, 1% BSA and 5% serum at room temperature for 1 hour and then incubated overnight at 4°C in a primary antibody solution diluted with a blocking reagent. The following antibodies were used as the primary antibodies: an anti-mouse Oct3/4 antibody (IgG; monoclonal; 1 : 300 dilution; Catalog No. sc-5279; Santa Cruz Biotechnology); an anti-mouse SSEA-4 antibody (IgG; monoclonal; 1: 300 dilution; Catalog No. MAB4304; Merck); and an anti-mouse SSEA-1 antibody (IgM; monoclonal; 1: 250 dilution; Catalog No. MAB4301; Merck).

Next, each cell type was washed twice with PBS and incubated at room temperature for 30 minutes with a secondary antibody solution diluted with a blocking reagent. The following antibodies were used as secondary antibodies: an anti-mouse IgG antibody (IgG; polyclonal; 1 : 300 dilution; Catalog No. A21202; Thermo Fisher Scientific) bound to Alexa Flour 488; and an IgG antibody (IgG; polyclonal; 1 : 300 dilution; Catalog No. A21207; Thermo Fisher Scientific) bound to anti-rabbit Alexa 594. Samples were each co-stained with 4',6-diamidino-2-phenylindole solution (DAPI, 1 : 1000 dilution; Catalog No. D523; Dojindo). Images were acquired using a BZ-9000 fluorescence microscope (KEYENCE CORPORATION) .

Human iPS cells and human iPS cells having a reduced undifferentiated level were fixed at room temperature for 15 minutes with 4% paraformaldehyde. After rinsing with PBS, the cells were incubated at room temperature for 1 hour with a 10 µg/mL FITC-bound rBC2LCN (Catalog No. 180-02991, Wako) solution prepared by dilution with PBS. Samples were each co-stained with a DAPI solution (1 : 1000 dilution; Catalog No. D523; Dojindo). Images were acquired using a BZ-9000 fluorescence microscope (KEYENCE CORPORATION) .

FIG. 1B depicts immunostaining images in the process of preparing human iPS cells and human iPS cells having a reduced undifferentiated level (cells on days 3 to 4 of culture). Human iPS cells were fluorescently stained green with the undifferentiation markers Oct3/4, rBC2-LCN, and SSEA-4, but were not stained with the SSEA-1 (Lewis X sugar chain antigen) antibody that reacts with differentiated human iPS cells. On the other hand, only some of human iPS cells having a reduced undifferentiated level prepared herein were stained with the undifferentiation markers (Oct3/4, rBC2-LCN, SSEA-4) and cells stained with the SSEA-1 antibody were confirmed.

The above results demonstrated that the human iPS cells having a reduced undifferentiated level included cells which were morphologically changed and stained with the SSEA-1 antibody.

### Example 2: Identification of SSEA-1 positive glycoprotein

A cell culture supernatant (100 µL) was incubated at room temperature for 3 hours with 90 µL of streptavidin-coated magnetic beads (Life Technologies) to which 9 µg of rABA lectin had been immobilized. After washing 5 times with 200 µL of PBST (PBS containing 1% Triton X-100), sample that had exhibited binding was eluted with 20 µL of 0.2% SDS at 95°C for 5 minutes. The eluted sample was electrophoresed under reducing conditions of 5-20% polyacrylamide gel (DRC). The isolated protein was transferred to a polyvinylidene difluoride (PVDF) membrane and incubated with a 1µg/mL HRP-labeled anti-mouse SSEA-1 monoclonal antibody (1 : 200, Catalog No. sc-21702; Santa Cruz Biotechnology) solution. Finally, detection was performed using Western Lighting Plus (PerkinElmer). Silver staining was performed using a Silver Staining MS kit (Catalog No. 293-77601, Wako Pure Chemical Industries, Ltd.).

FIG. 2A depicts the results of immunoprecipitation and Western blotting performed using each culture supernatant of human iPS cells (control) and human iPS cells having a reduced undifferentiated level. When a mucin-like glycoprotein was concentrated from each culture supernatant using rABA lectin that reacts with a mucin-like glycoprotein and then cells were stained with the SSEA-1 antibody, a > 250 kDa band (indicated by an arrow) was observed in the culture supernatant of human iPS cells having a reduced undifferentiated level. On the other hand, no > 250 kDa band was observed in the culture supernatant of human iPS cells.

FIG. 2B depicts the results of silver staining of samples subjected to immunoprecipitation obtained using the culture supernatant of human iPS cells having a reduced undifferentiated level and that of human iPS cells. A > 250 kDa band (indicated by an arrow) observed in the culture supernatant of human iPS cells having a reduced undifferentiated level was used for LC-MS/MS analysis.

In LC-MS/MS analysis, an analysis region (indicated by an arrow) of the culture supernatant of human iPS cells having a reduced undifferentiated level was excised from SDS gel after silver staining. As a control, the corresponding region in the culture supernatant of human iPS cells was also excised from the SDS gel. Digestion in the gel was carried out according to a conventionally known method (Shevchenko et al., Anal. Chem. 1996). Specifically, the gel pieces were washed with water and acetonitrile, and then each sample was alkylated with dithiothreitol (DTT) and iodoacetamide. Trypsin was then added and then the resultant was maintained at 37°C for 16 hours for hydrolysis reaction. After the reaction, peptides were extracted from the gel pieces using 25 mM ammonium hydrogen carbonate, 5% formic acid, and acetonitrile. Finally, the extraction solution was dried under reduced pressure.

Next, the dried peptide sample was dissolved in a solvent consisting of water, acetonitrile and trifluoroacetic acid (volume ratio 98:2:0.1) and then subjected to LC-MS/MS analysis. LC-MS/MS analysis was performed using a paradigm MS4 liquid chromatograph (Michrom Bioresources) and an LTQ OrbiTrap XL mass spectrometer (Thermo Fisher Scientific).

The MS/MS data acquired by LC-MS/MS analysis was subjected to sequence database search. Mascot (ver. 2.5) (http://www.matrixscience.com/) manufactured by Matrix Science was used as the search software. The sequence data set for search was constructed by adding the amino acid sequence of swine (Sus scrofa) trypsin to protein entries (20,192 in total) derived from human (Homo sapiens) output from the SwissProt (http://www.uniprot.org/) 2017_08 version. The search result was input to a browsing software Scaffold (Proteome Software). Proteins each identified based on two or more unique peptides with a Mascot ion score equal to or above the identity score threshold were considered significant. FIG. 2C lists the thus identified proteins. As a result, it was found that the most frequently detected peptide in the analysis region (indicated by an arrow) of the culture supernatant of human iPS cells having a reduced undifferentiated level was fibronectin. On the other hand, fibronectin was not detected in the corresponding region of the culture supernatant of human iPS cells.

### Example 3: Detection of SSEA-1-positive fibronectin in the culture supernatant of human iPS cells having a reduced undifferentiated level

### 1. Immunoprecipitation and Western blotting

A cell culture supernatant (95 µL) was incubated at room temperature for 3 hours using 40 µL of streptavidin-coated magnetic beads (Life Technologies) on which 4 µg of a biotin-labeled fibronectin polyclonal antibody (Catalog No. AF1918; R & D) had been immobilized. After washing 5 times with 200 µL of PBST (PBS containing 1% Triton X-100), sample that had exhibited binding was eluted with 20 µL of 0.2% SDS at 95°C for 5 minutes. The eluted sample was electrophoresed under reducing conditions of 5-20% polyacrylamide gel (DRC). The isolated protein was transferred to a polyvinylidene difluoride (PVDF) membrane and then incubated with a 1 µg/mL HRP-labeled anti-mouse SSEA-1 monoclonal antibody (1: 200, Catalog No. sc-21702; Santa Cruz Biotechnology) solution or a fibronectin polyclonal antibody (Catalog No. AF1918; R & D). In the case of the fibronectin polyclonal antibody, an HRP-labeled anti-sheep IgG antibody (Catalog No. 313-035-003; Jackson ImmunoResearch) was used for the secondary antibody reaction. Finally, detection was performed using Western Lighting Plus-ECL (PerkinElmer).

Further, a cell culture supernatant (95 µL) was incubated at room temperature for 3 hours using 40 µL of streptavidin-coated magnetic beads (Life Technologies) on which 4 µg of a biotin-labeled fibronectin monoclonal antibody (Catalog No. MAB1918; R & D) had been immobilized. After washing 5 times with 200 µL of PBST (PBS containing 1% Triton X-100), sample that had exhibited binding was eluted with 20 µL of 0.2% SDS at 95°C for 5 minutes. The eluted sample was electrophoresed under reducing conditions of 5-20% polyacrylamide gel (DRC). The isolated protein was transferred to a polyvinylidene difluoride (PVDF) membrane and then incubated with a 1 µg/mL HRP-labeled anti-mouse SSEA-1 monoclonal antibody (1 : 200, Catalog No. sc-21702; Santa Cruz Biotechnology) solution or a fibronectin monoclonal antibody (Catalog No. MAB1918; R & D). In the case of the fibronectin polyclonal antibody, an HRP-labeled anti-mouse IgG antibody (Catalog No. 115-035-003; Jackson ImmunoResearch) was used for the secondary antibody reaction. Finally, detection was performed using Western Lighting Plus (PerkinElmer).

### 2. Immunostaining

Human iPS cells (control) and human iPS cells having a reduced undifferentiated level were each fixed for 15 minutes at room temperature with 4% paraformaldehyde (Catalog No. 163-20145; Wako) and then rinsed with PBS. The cells were pre-hybridized at room temperature for 1 hour in PBS containing a blocking reagent, 1% BSA and 5% serum and then incubated at 4°C overnight in an antibody solution diluted with a blocking reagent. The following antibody was used: biotin-labeled fibronectin monoclonal antibody (1 : 150; Catalog No. MAB1918; R & D).

Next, each cell type was washed twice with PBS and incubated at room temperature for 30 minutes with PE-labeled avidin (1 : 100 dilution; Catalog No. 016-110-084; Jackson ImmunoResearch) diluted with a blocking reagent. Samples were co-stained with a DAPI solution (1 : 1000 dilution; Catalog No. D523; Dojindo). Images were acquired using a BZ-9000 fluorescence microscope (KEYENCE CORPORATION).

FIGS. 3A and 3B depict the results of immunoprecipitation and Western blotting performed using each culture supernatant of human iPS cells (control) and human iPS cells having a reduced undifferentiated level. A > 250 kDa band was detected in the culture supernatant of human iPS cells having a reduced undifferentiated level (indicated by an arrow). On the other hand, no > 250 kDa band was detected in the culture supernatant of human iPS cells. Therefore, it was found that the SSEA-1 antibody-positive > 250 Da glycoprotein was fibronectin that was secreted in the culture supernatant of human iPS cells having a reduced undifferentiated level.

FIG. 3C depicts immunostaining images of human iPS cells and human iPS cells having a reduced undifferentiated level. Although human iPS cells were not stained for fibronectin, human iPS cells having a reduced undifferentiated level were stained for fibronectin.

### Example 4: Sandwich ELISA assay using fibronectin antibody and SSEA-1 antibody

A biotin-labeled human fibronectin monoclonal antibody (Catalog No. FN 30-8, Takara Bio) was immobilized on the surface of an avidin-coated microplate well (Catalog No. BS-X7603, Sumitomo Bakelite). Each culture supernatant of human iPS cells (control) and human iPS cells having a reduced undifferentiated level was diluted in such a manner that the concentration was a predetermined value and reacted at room temperature for 1 hour. Subsequently, an HRP-labeled SSEA-1 monoclonal antibody (Catalog No. sc-21702; Santa Cruz Biotechnology) was reacted. After washing with 1 × PBS containing 0.1% (v/v) Tween 20, samples were each incubated with a TMB solution (Catalog No. 208-17371, Wako) at room temperature for 30 minutes. The enzyme reaction was stopped by adding 1N HCl and then the enzyme activity on the microplate was measured at a main wavelength of 450 and a sub-wavelength of 620 nm (OD450/620) using a microtiter plate reader.

FIG. 4A depicts the results of constructing sandwich ELISA using a fibronectin antibody and an SSEA-1 antibody and then analyzing each culture supernatant of human iPS cells and human iPS cells having a reduced undifferentiated level at each dilution stage. As a result, it was found that although high reactivity to the culture supernatant (black circles) of human iPS cells having a reduced undifferentiated level was observed, no reactivity to the culture supernatant (white triangles) of human iPS cells was observed. It was found that the lower detection limit of human iPS cells having a reduced undifferentiated level was 100 cells/mL, by which even a small number of human iPS cells having a reduced undifferentiated level can be detected. Therefore, it was found that through detection of SSEA-1-positive fibronectin using a culture supernatant, human iPS cells whose undifferentiated level has been reduced or disappeared can be detected non-invasively and with high sensitivity.

FIG. 4B is a graph showing the results of sandwich ELISA using the culture supernatants when human iPS cells having a reduced undifferentiated level were seeded at 25,000 cells/mL, 12,500 cells/mL, 6,250 cells/mL, and 0 cells/mL, respectively. Black indicates the actual number of cells, gray indicates the apparent number of cells when human iPS cells having a reduced undifferentiated level were cultured alone, and white indicates the apparent number of cells when the cells were co-cultured with human iPS cells. It was found that the number of cells calculated by the sandwich ELISA method exhibited reactivity depending on the number of seeded cells and was almost the same as the actual number of cells. Furthermore, it was found that deviant cells can be detected even in the coexistence of human iPS cells. It was assumed that the reason why the slightly higher value was confirmed in the coexistence of human iPS cells was that deviant cells were mixed in the seeded human iPS cells.

### Example 5: Sandwich ELISA assay using fibronectin monoclonal antibody and fibronectin polyclonal antibody

A biotin-labeled human fibronectin monoclonal antibody (Catalog No. FN 30-8, Takara Bio) was immobilized on the surface of an avidin-coated microplate well (Catalog No. BS-X7603, Sumitomo Bakelite). Each culture supernatant of human iPS cells (control) and human iPS cells having a reduced undifferentiated level was diluted in such a manner that the concentration was a predetermined value and reacted at room temperature for 1 hour. Subsequently, an HRP-labeled fibronectin polyclonal antibody (Catalog No. AF1918; R & D) was reacted. After washing with 1 × PBS containing 0.1% (v/v) Tween 20, the samples were each incubated with a TMB solution (Catalog No. 208-17371, Wako) for 30 minutes at room temperature. The enzyme reaction was stopped by adding 1N HCl and the enzyme activity on the microplate was measured at a main wavelength of 450 and a sub-wavelength of 620 nm (OD450/620) using a microtiter plate reader.

A sandwich ELISA was constructed in the same manner as in Example 4 above except the use of the fibronectin monoclonal antibody and the fibronectin polyclonal antibody. FIG. 5 depicts the results of analyzing each culture supernatant of human iPS cells and human iPS cells having a reduced undifferentiated level at each dilution stage using the sandwich ELISA. As a result, it was found that although high reactivity was exhibited to the culture supernatant (black circles) of human iPS cells having a reduced undifferentiated level, no reactivity was exhibited to the culture supernatant (white triangles) of human iPS cells. It was found that the lower detection limit of human iPS cells having a reduced undifferentiated level was 100 cells/mL, by which even a small number of human iPS cells having a reduced undifferentiated level can be detected. Therefore, it was found that through detection of fibronectin using a culture supernatant, human iPS cells whose undifferentiated level has been reduced or disappeared can be detected non-invasively and with high sensitivity.

### Example 6: Living cell imaging of human iPS cells (control) and human iPS cells having a reduced undifferentiated level with IR700-labeled fibronectin antibody

IR700 labeling was performed using an IRDye700DX protein labeling kit (LI-COR Inc). A fibronectin antibody (Catalog No. AF1918; R & D) or a polyclonal antibody against sheep immunoglobulin G (Catalog No. 5-001-A; R & D) was incubated with IR700 at room temperature for 2 hours. The molar ratio and concentration of a protein were determined by measuring the absorbance at 280 nm (protein) and 689 nm (IR700), respectively, using a UV-1900 spectrophotometer (Shimadzu).

Cells were incubated with the IR700-labeled fibronectin antibody at 5% CO₂ and 37°C for 1 hour or 6 hours. For nuclear staining, cells were further incubated with a Hoechst 33342 solution (1 : 1000 dilution, Dojindo Molecular Technologies, Inc) for 5 to 10 minutes. Images were acquired with a confocal microscope (Olympus Corporation) or a BZ-9000 fluorescence microscope (KEYENCE CORPORATION).

FIG. 6A depicts immunostaining images of living human iPS cells (control) and living human iPS cells having a reduced undifferentiated level stained with the IR700-labeled fibronectin antibody (red) at room temperature for 1 hour. The nuclei were stained with Hoechst 33342 (blue). The scale bar indicates 100 µm. In addition, FIG. 6B depicts immunostaining images of living human iPS cells having a reduced undifferentiated level stained with the IR700-labeled fibronectin antibody (red) at 37°C for 1 hour (left panel) or 6 hours (right panel). The scale bar indicates 100 µm.

Binding of the IR700-labeled fibronectin antibody to human iPS cells having a reduced undifferentiated level was confirmed, but no such binding to human iPS cells was confirmed. No difference was confirmed in the amount of binding of the human iPS cells having a reduced undifferentiated level to the IR700-labeled fibronectin antibody between the co-incubation time of 1 hour and that of 6 hours (the binding was maintained sufficiently even after 6 hours).

### Example 7: Verification of the effect of IR700-labeled fibronectin antibody and near-infrared (NIR) radiation on the cell viability of human iPS cells (control) and human iPS cells having a reduced undifferentiated level

The day before NIR radiation, cells were seeded on 96-well or 12-well plates and incubated for 24 hours. After medium exchange, the cells were incubated with an IR700-labeled fibronectin antibody or IR700-labeled sheep immunoglobulin G for 1 hour or 6 hours, and then irradiated with NIR light-emitting diodes at a power density of 10 mW/cm² (Ebisudenshi Co., Ltd.). Images were acquired using a BZ-9000 fluorescence microscope (KEYENCE CORPORATION).

Cell viability was analyzed using a LIVE/DEAD Viability/Cytotoxicity Kit (Thermo Fisher Scientific) for mammalian cells. Living cells were stained with 2 µM calcein-acetoxymethyl (AM) and dead cells were stained with 4 µM ethidium homodimer 1 (EthD-1) at 37°C for 40 minutes. Images were acquired using a BZ-9000 fluorescence microscope (KEYENCE CORPORATION).

FIG. 7A depicts the results of subjecting human iPS cells having a reduced undifferentiated level to no treatment, treatment with the IR700-labeled fibronectin antibody, and NIR radiation treatment, and treatment with the IR700-labeled fibronectin antibody followed by NIR radiation, respectively, and then analyzing the cell viability by living cell staining (calcein-AM: green) and dead cell staining (EthD-1: red). Almost no dead cells were observed among cells subjected to no treatment, treatment with the IR700-labeled fibronectin antibody, and NIR radiation, but increases in dead cells and decreases in living cells were confirmed for cells subjected to treatment with the IR700-labeled fibronectin antibody followed by NIR radiation.

FIG. 7B depicts the results of subjecting human iPS cells (controls) to no treatment, treatment with the IR700-labeled fibronectin antibody, NIR radiation, and treatment with the IR700-labeled fibronectin antibody followed by NIR radiation, respectively, and then analyzing the cell viability by living cell staining (calcein-AM: green) and dead cell staining (EthD-1: red). Unlike the above human iPS cells having a reduced undifferentiated level, almost no dead cells were observed among cells subjected to treatment with the IR700-labeled fibronectin antibody followed by NIR radiation.

### Example 8: Quantitative effects of IR700-labeled fibronectin antibody and NIR radiation on the cell viability of human iPS cells (control) and human iPS cells having a reduced undifferentiated level

Cell viability was quantified using Cell-Counting Kit-8 (Dojindo Molecular Technologies, Inc). Ten (10) µL of Cell-Counting Kit-8 solution was added to cells in each well of a 96-well plate and incubated at 37°C for 2 hours. Then, the absorbance at 450 nm was measured using a microtiter plate reader (SpectraMax M3, Molecular Devices). For calculation of cell viability, an untreated medium incubated at 37°C for 2 hours was used as a negative control.

Human iPS cells (control) and human iPS cells having a reduced undifferentiated level were incubated with various concentrations of an IR700-labeled fibronectin antibody (0 to 10 ng/mL) for 1 hour, and then exposed to NIR radiation for 10 minutes. After 24 hours, 10 µL of CCK-8 solution was added to the medium, and after further incubation for 2 hours, the absorbance at 450 nm was measured. FIG. 8A depicts each cell viability (%) of human iPS cells (control) (white circles) and human iPS cells (black circles) having a reduced undifferentiated level. Cell viability (%) was calculated by comparing the absorbance at 450 nm of cells subjected to treatment with the IR700-labeled fibronectin antibody followed by NIR radiation with that of untreated cells. The data are represented by the average value in triplicate measurements ± standard deviation. As a result, it was confirmed that the cell viability of human iPS cells having a reduced undifferentiated level can be significantly decreased by the treatment with the IR700-labeled fibronectin antibody followed by NIR radiation. Further, the effect was obtained by the use of the IR700-labeled fibronectin antibody in an amount as small as about 1 ng/mL, and when the IR700-labeled fibronectin antibody was used in an amount of about 10 ng/mL, the cells were almost killed.

Further, human iPS cells (control) and human iPS cells having a reduced undifferentiated level were each incubated with the 10 ng/mL IR700-labeled fibronectin antibody for 1 hour, and then exposed to NIR radiation for various times (0 to 10 minutes). After 24 hours, 10 µL of CCK-8 solution was added to the medium, further incubation was performed for 2 hours, and then the absorbance at 450 nm was measured. FIG. 8B depicts each cell viability (%) of human iPS cells (control) (white circles) and human iPS cells (black circles) having a reduced undifferentiated level. Cell viability (%) was calculated by comparing the absorbance at 450 nm of cells subjected to treatment with the IR700-labeled fibronectin antibody alone with that of cells subjected to treatment with the IR700-labeled fibronectin antibody followed by NIR radiation. The data are represented by the average value in triplicate measurements ± standard deviation. As a result, it was confirmed that the cell viability of human iPS cells having a reduced undifferentiated level treated with the IR700-labeled fibronectin antibody was significantly reduced by brief NIR radiation of about 3 minutes. About 5 minutes of irradiation completely killed the cells.

### Example 9: Selective removal of human iPS cells having a reduced undifferentiated level from culture with human iPS cells (control) by treatment with IR700-labeled fibronectin antibody and NIR radiation

FIG. 9A depicts the results of subjecting human iPS cells (control) and human iPS cells having a reduced undifferentiated level to no treatment, treatment with an IR700-labeled fibronectin antibody, NIR radiation, and treatment with the IR700-labeled fibronectin antibody followed by NIR radiation, respectively, and then analyzing the co-culture of the human iPS cells (long elongated large flat shape) having a reduced undifferentiated level with the control human iPS cells (colony forming cells) by living cell staining (calcein-AM: green) and dead cell staining (EthD-1: red). As a result, increases in dead cells and decreases in living cells were confirmed in the human iPS cells having a reduced undifferentiated level subjected to treatment with the IR700-labeled fibronectin antibody followed by NIR radiation.

Furthermore, adhered human iPS cells having a reduced undifferentiated level were stained with 20 µM CellTrackerGreen-5-chloromethylfluorescein diacetate (CMFDA) (Thermo Fisher Scientific) and then co-cultured with human iPS cells (control) in wells of 12-well plates. The resultant was subjected to no treatment, treatment with the IR700-labeled fibronectin antibody, NIR radiation, and treatment with the IR700-labeled fibronectin antibody followed by NIR radiation, respectively. Then a single cell suspension containing human iPS cells (control) and human iPS cells having a reduced undifferentiated level was prepared using a TrypLE Express recombinant enzyme solution (ThermoFisher Scientific) and then suspended in phosphate buffered saline (PBS) containing 1% bovine serum albumin. The thus obtained cell suspension was subjected to data analysis using a CytoFLEX flow cytometer (Beckman Coulter Inc.) and FlowJov10 software (BDBiosciences), and the abundance of each cell type was confirmed. FIG. 9B depicts the analysis results. As a result, it was confirmed that human iPS cells having a reduced undifferentiated level was selectively removed from the co-culture by treatment with the IR700-labeled fibronectin antibody followed by NIR radiation.

### Industrial Applicability

According to the present invention, the undifferentiated state of human iPS cells can be determined or evaluated non-invasively, simply and efficiently without reducing the number of cells, and only cells having a low undifferentiated level can be selectively removed. Therefore, the present invention is an extremely useful technique that is expected to contribute to the quality control of human iPS cells.

## Claims

1. A method for controlling the quality of human pluripotent stem cells, comprising a step of detecting or measuring an SSEA-1-positive fibronectin in a culture supernatant of the human pluripotent stem cells to determine or evaluate undifferentiated state of the human pluripotent stem cells,
wherein the detection or measurement results of SSEA-1 positive fibronectin in the culture supernatant of the human pluripotent stem cells correlates with the undifferentiated state of the human pluripotent stem cells.

2. The method according to claim 1, wherein the step of detecting or measuring the SSEA-1-positive fibronectin in the culture supernatant of the human pluripotent stem cells is performed using (a) a probe that recognizes an SSEA-1 as an epitope and (b) a probe that recognizes the fibronectin.

3. The method according to claim 2, wherein (a) the probe that recognizes the SSEA-1 as the epitope is an anti SSEA-1 antibody or a fragment thereof and/or (b) the probe that recognizes the fibronectin is an anti fibronectin antibody or a fragment thereof.

4. The method according to any one of claims 1 to 3, wherein the step of detecting or measuring the SSEA-1-positive fibronectin in the culture supernatant of the human pluripotent stem cells is performed by a sandwich assay method using the probes of (a) and (b).

5. The method according to any one of claims 1 to 4, further comprising a step of collecting the human pluripotent stem cells, a step of removing cells having a low undifferentiated level from the culture that the SSEA-1-positive fibronectin is detected in the culture supernatant, or a step of discarding the culture.

6. Use of a kit in a method for controlling the quality of human pluripotent stem cells, the kit comprising (a) a probe that recognizes an SSEA-1 as an epitope and (b) a probe that recognizes a fibronectin.

7. The use according to claim 6, wherein(a) the probe that recognizes the SSEA-1 as the epitope is an anti SSEA-1 antibody or a fragment thereof and/or (b) the probe that recognizes the fibronectin is an anti fibronectin antibody or a fragment thereof.

8. The use according to claim 6 or 7, wherein one probe of either (a) or (b) is immobilized on a substrate and the other probe is labeled.

## Patentansprüche

1. Verfahren zum Kontrollieren der Qualität von humanen pluripotenten Stammzellen, umfassend einen Schritt zum Detektieren oder Messen eines SSEA-1-positiven Fibronektins in einem Kulturüberstand der humanen pluripotenten Stammzellen, um den undifferenzierten Zustand der humanen pluripotenten Stammzellen zu bestimmen oder zu bewerten,
wobei die Detektions- oder Messergebnisse von SSEA-1-positivem Fibronektin im Kulturüberstand der humanen pluripotenten Stammzellen mit dem undifferenzierten Zustand der humanen pluripotenten Stammzellen korrelieren.

2. Verfahren gemäß Anspruch 1, wobei der Schritt des Detektierens oder Messens des SSEA-1-positiven Fibronektins im Kulturüberstand der humanen pluripotenten Stammzellen unter Verwendung (a) einer Sonde ("probe"), die ein SSEA-1 als ein Epitop erkennt, und (b) einer Sonde, die das Fibronektin erkennt, durchgeführt wird.

3. Verfahren gemäß Anspruch 2, wobei (a) die Sonde, die SSEA-1 als das Epitop erkennt, ein Anti-SSEA-1-Antikörper oder ein Fragment davon ist und/oder (b) die Sonde, die das Fibronektin erkennt, ein Anti-Fibronektin-Antikörper oder ein Fragment davon ist.

4. Verfahren gemäß irgendeinem der Ansprüche 1 bis 3, wobei der Schritt des Detektierens oder Messens des SSEA-1-positiven Fibronektins im Kulturüberstand der humanen pluripotenten Stammzellen durch ein Sandwich-Assay-Verfahren unter Verwendung der Sonden aus (a) und (b) durchgeführt wird.

5. Verfahren gemäß irgendeinem der Ansprüche 1 bis 4, ferner umfassend einen Schritt des Sammelns der humanen pluripotenten Stammzellen, einen Schritt des Entfernens von Zellen, die eine niedriges undifferenziertes Level haben, aus der Kultur, bei der SSEA-1-positives Fibronektin im Kulturüberstand nachgewiesen wird, oder einen Schritt des Verwerfens der Kultur.

6. Verwendung eines Kits in einem Verfahren zum Kontrollieren der Qualität von humanen pluripotenten Stammzellen, das Kit umfassend (a) eine Sonde, die ein SSEA-1 als ein Epitop erkennt, und (b) eine Sonde, die ein Fibronektin erkennt.

7. Verwendung gemäß Anspruch 6, wobei (a) die Sonde, die das SSEA-1 als das Epitop erkennt, ein Anti-SSEA-1-Antikörper oder ein Fragment davon ist und/oder (b) die Sonde, die das Fibronektin erkennt, ein Anti-Fibronektin-Antikörper oder ein Fragment davon ist.

8. Verwendung gemäß Anspruch 6 oder 7, wobei eine Sonde aus (a) oder (b) auf einem Substrat immobilisiert ist und die andere Sonde markiert ist.

## Revendications

1. Procédé de contrôle de la qualité de cellules souches pluripotentes humaines, comprenant une étape de détection ou de mesurage d'une fibronectine positive à SSEA-1 dans un surnageant de culture des cellules souches pluripotentes humaines pour déterminer ou évaluer un état indifférencié des cellules souches pluripotentes humaines,
dans lequel les résultats de détection ou de mesure de la fibronectine positive à SSEA-1 dans le surnageant de culture des cellules souches pluripotentes humaines sont en corrélation avec l'état indifférencié des cellules souches pluripotentes humaines.

2. Procédé selon la revendication 1, dans lequel l'étape de détection ou de mesure de la fibronectine positive à SSEA-1 dans le surnageant de culture des cellules souches pluripotentes humaines est mise en œuvre en utilisant (a) une sonde qui reconnaît un SSEA-1 en tant qu'un épitope et (b) une sonde qui reconnaît la fibronectine.

3. Procédé selon la revendication 2, dans lequel (a) la sonde qui reconnaît le SSEA-1 en tant que l'épitope est un anticorps anti-SSEA-1 ou un fragment de celui-ci et/ou (b) la sonde qui reconnaît la fibronectine est un anticorps anti-fibronectine ou un fragment de celui-ci.

4. Procédé selon l'une quelconque des revendications 1 à 3, dans lequel l'étape de détection ou de mesure de la fibronectine positive à SSEA-1 dans le surnageant de culture des cellules souches pluripotentes humaines est mise en œuvre par un procédé d'essai en sandwich utilisant les sondes de (a) et (b).

5. Procédé selon l'une quelconque des revendications 1 à 4, comprenant en outre une étape de collecte des cellules souches pluripotentes humaines, une étape d'élimination de cellules présentant un faible niveau indifférencié de la culture où la fibronectine positive à SSEA-1 est détectée dans le surnageant de culture, ou une étape de mise au rebut de la culture.

6. Utilisation d'un kit dans un procédé de contrôle de la qualité de cellules souches pluripotentes humaines, le kit comprenant (a) une sonde qui reconnaît un SSEA-1 en tant qu'un épitope et (b) une sonde qui reconnaît une fibronectine.

7. Utilisation selon la revendication 6, dans laquelle (a) la sonde qui reconnaît le SSEA-1 en tant que l'épitope est un anticorps anti-SSEA-1 ou un fragment de celui-ci et/ou (b) la sonde qui reconnaît la fibronectine est un anticorps anti-fibronectine ou un fragment de celui-ci.

8. Utilisation selon la revendication 6 ou la revendication 7, dans laquelle une sonde de soit (a) soit (b) est immobilisée sur un substrat et l'autre sonde est étiquetée.
